# FASCICULE DE BREVET EUROPEEN

(11) **EP 3 975 947 B1**
(45) Date de publication et mention de la délivrance du brevet: **09.08.2023**
(21) Numéro de dépôt: 19739676.5
(22) Date de dépôt: 28.05.2019
(51) Int. Cl.: A61F 6/02

(54) **DISPOSITIF ANNULAIRE A SURFACE INTERNE D'ADHÉRENCE, FABRICATION ET UTILISATION POUR UNE CONTRACEPTION MASCULINE DU DISPOSITIF ANNULAIRE**
RINGFÖRMIGE VORRICHTUNG MIT INNERER ADHÄSIONSFLÄCHE, HERSTELLUNG UND VERWENDUNG DER RINGFÖRMIGEN VORRICHTUNG FÜR EIN MÄNNLICHES EMPFÄNGNISVERHÜTUNGSMITTEL
ANNULAR DEVICE WITH INNER ADHESION SURFACE, MANUFACTURE AND USE OF THE ANNULAR DEVICE FOR A MALE CONTRACEPTIVE

(43) Date de publication de la demande: 06.04.2022
(73) Titulaire: Labrit, Maxime, 33160 Saint-Médard-en-Jalles (FR)
(72) Inventeur: Labrit, Maxime, 33160 Saint-Médard-en-Jalles (FR)
(74) Mandataire: Plasseraud IP
(86) Numéro de dépôt international: PCT/FR2019/051254
(87) Numéro de publication internationale: WO 2020/240101

(56) Documents cités:
- DE-U1- 29 910 215
- FR-A1- 2 772 260
- FR-B1- 2 772 260
- US-A1- 2010 179 379
- US-A1- 2014 224 263
- US-A1- 2016 106 569
- US-A1- 2017 135 895

## Description

La présente invention concerne le domaine des dispositifs de contraception masculine et relève de la catégorie A61F 6/02 (« *Dispositif de contraception à usage masculin* ») au sens de la classification internationale des brevets. L'invention concerne plus particulièrement un dispositif annulaire, qui ne requiert ni prise d'hormones ni intervention chirurgicale, pour permettre d'obtenir un effet contraceptif chez l'homme. L'invention se rapporte également à une utilisation de ce dispositif annulaire et à son procédé de fabrication.

On connaît, par exemple par le document US 5063939 A, un dispositif de contraception par augmentation de la température dans le scrotum. Ce dispositif consiste en une poche chauffante recevant le scrotum et qui s'accroche au pénis par une paire de bandes d'attache. L'effet de contraception est obtenu sur la base du principe que la chaleur stoppe la spermatogénèse. La poche a une structure à double paroi, permettant d'insérer un élément chauffant raccordé électriquement à une source d'alimentation.

Ce type de solution n'est pas ergonomique et le manque de confort rend le dispositif peu attrayant. En outre, la présence d'un élément chauffant électrique présente certains risques pour l'utilisateur. Il faut également constater que l'utilisation de slips chauffants, comme illustré notamment par le document CN 103919284 A, n'a pas connu d'engouement. Le document DE 29910215 U1 propose un dispositif annulaire à section en V, visant à favoriser le maintien en position haute des testicules de l'utilisateur, qui sont chauffées par la chaleur corporelle. Bien que compatible avec une certaine liberté de mouvement, ce type de dispositif correspond aussi à une conception limitant le confort.

Une autre méthode de réchauffement des testicules est la prise de bains chauds dans lesquels les testicules sont immergés dans de l'eau chaude de façon régulière. Cette méthode n'est pas commode et est chronophage car elle implique une procédure compliquée avec une structure complexe. De plus, elle n'évite pas le risque de brûlure.

Il reste ainsi un besoin d'améliorer les dispositifs permettant un effet de contraception masculine, en termes d'ergonomie et de confort, tout en présentant une efficacité pour éviter d'obtenir les conditions de température favorables à la spermatogénèse.

Le préservatif correspond à une solution répandue pour la contraception. Cependant, il constitue un produit jetable relativement coûteux et écologiquement discutable. En outre, le taux d'échec n'est pas négligeable, ce qui peut être attribué au fait qu'il peut glisser de façon inaperçue ou se déchirer. Il reste vulnérable aux risques d'endommagements (en combinaison avec certains contraceptifs chimiques appliqués de l'extérieur ou du fait de manipulations qui agressent le matériau). Par ailleurs, le préservatif interfère dans l'acte sexuel et peut avoir un effet bloquant ou atténuant les sensations.

Des dispositifs sont par ailleurs connus pour des applications de maintien d'érection, ce qui relève de la catégorie A61F 5/41 au sens de la classification internationale des brevets, par exemple FR 2 772 260, US 2016/106569 ou encore US 2010/179379. De tels dispositifs, qui ne doivent être portés que le temps d'une érection, ne conviennent pas pour une contraception thermique (un effet de contraception impliquant une durée de port importante, sans commune mesure avec la durée de port d'un dispositif de maintien ou stimulation d'érection).En vue d'améliorer la situation, l'invention concerne un dispositif de contraception masculine adapté pour encercler une verge en étant de forme générale annulaire autour d'un axe longitudinal virtuel, le dispositif comprenant :
- deux bords annulaires formant deux extrémités axiales opposées du dispositif, un premier bord dit arrière de ces deux bords étant adapté pour venir buter du côté de la racine de la verge à l'état monté du dispositif avec la verge encerclée ;
- une face externe ;
- une face interne délimitant un volume intérieur et présentant des reliefs pour former une surface interne d'adhérence du dispositif, la face interne comprenant une portion de surface supérieure, préférentiellement concave, destinée à être en contact avec la verge du côté de sa veine dorsale superficielle ;
- une portion de surface pour le contact scrotal adaptée pour être espacée de la verge et située à l'opposé de la portion de surface supérieure, de sorte qu'elle permet de maintenir une partie de peau scrotale entre la verge placée dans le volume intérieur et ladite portion de surface, tandis que ledit bord arrière repousse les testicules en arrière du volume intérieur typiquement à distance de ce volume intérieur, le maintien en arrière correspondant aussi à un effet de remontée des testicules qui ne peuvent plus descendre au fond du scrotum (et qui *de facto* remontent au-dessus par rapport à l'axe de l'anneau).

Typiquement, la portion de surface est formée au moins pour partie par la surface interne d'adhérence et/ou par des moyens de retenue formant une fente qui s'étend sous la surface interne d'adhérence.

La partie de peau scrotale peut être placée et maintenue entre la verge placée dans le volume intérieur et la portion de surface avec un effet de retenue qui n'est pas inconfortable, pour un effet de contraception masculine thermique à l'état monté du dispositif avec la verge et la peau scrotale du scrotum vide encerclées.

Un très bon compromis entre confort et retenue de la peau scrotale est obtenu avec des reliefs resserrés, en particulier sur portion de surface (inférieure) pour le contact contre la peau scrotale. Il est ainsi préféré de disposer d'au moins 8 reliefs appartenant à une même trajectoire circulaire dans une coupe transversale du dispositif annulaire, sur une moitié (demi circonférence, c'est-à-dire sur un demi-anneau) pour le contact avec la peau scrotale. On comprend qu'une série d'au moins 15 ou 16 reliefs sur une telle moitié d'anneau (alignement de 15 ou 16 reliefs sur une trajectoire généralement demi-circulaire) peut être prévue pour densifier les points de contact avec effet de retenue. Ces reliefs, qui sont en saillie vers l'intérieur, permettent de multiplier des zones de contact discrètes/discontinues dont l'écartement est réduit. De préférence, au moins 20 ou 25 reliefs sont répartis sur une circonférence complète selon une vue en coupe transversale de l'anneau.

Dans une forme de réalisation préférentielle, les deux bords annulaires, la face externe et la face interne sont formés par un corps, typiquement réalisé d'une seule pièce. Lorsqu'il est prévu des moyens de retenue formant une fente, ceux peuvent être aussi formés par ce corps en matière élastiquement déformable. Alternativement, de tels moyens de retenue peuvent être rapportés sur le corps, typiquement du côté de la partie inférieure.

Grâce à ces dispositions, en particulier avec des reliefs, le dispositif forme un anneau pénoscrotal, la face interne exerçant une action de maintien de la partie de peau scrotale dans une conformation d'encerclement à la fois de la verge et du scrotum vide. Le dispositif permet de déplacer et remonter les testicules de façon commode, en faisant glisser vers l'arrière la face interne contre la verge et en intercalant en sens inverse le scrotum vide entre la verge et la surface interne d'adhérence. Le bord arrière du dispositif fait lors barrage à la descente des testicules qui restent proches du canal inguinal. Les reliefs permettent d'améliorer le maintien des testicules en position supra-scrotale pour induire une élévation de la température des testicules avec le corps comme seule source de chaleur, en limitant le glissement de l'anneau lors de la mobilisation (marche, course, saut, etc.). Ils améliorent la tenue générale de l'anneau à la racine de la verge.

Chez l'homme l'induction d'une élévation de quelques degrés de la température des testicules maintenue sur une longue période et répétée quotidiennement entraîne une réduction de la production de spermatozoïdes et de leur mobilité. Le dispositif permet de déplacer les testicules, du scrotum à la racine de la verge à proximité de l'orifice externe du canal inguinal, c'est-à-dire dans une zone plus chaude. L'utilisation reste simple puisque le placement à l'intérieur de l'anneau du pénis et du scrotum sans les testicules est une opération rapide.

La réversibilité de la méthode (mise en infertilité provisoire / maîtrisée individuellement), l'absence d'effets secondaires sont en outre des avantages significatifs.

De préférence, le dispositif est sous la forme d'un anneau et le matériau constitutif de la face interne, souple et déformable, permet de faire glisser l'anneau autour de la verge et du scrotum, sans effet de striction sur la verge, pour que l'anneau soit au contact par son bord arrière contre la zone abdominale de l'aine.

Dans une option particulière, le dispositif est essentiellement réalisé en élastomère, typiquement un matériau silicone. Ce genre de matériau souple mais adhérant, la présence des reliefs spécifiques dans un format d'anneau (dont le diamètre peut être prédéterminé en fonction d'une taille adaptée, par exemple à choisir entre au moins trois tailles : 30 mm, 40 mm et 50 mm de diamètre ou 33, 38 et 43 mm, à titre d'exemples non limitatifs), permettent de maintenir en place le dispositif, garantissant une stabilité des testicules en position optimale d'induction d'une faible élévation de chaleur avec comme seule source celle du corps de l'utilisateur et en évitant de provoquer une striction pénienne ou une quelconque altération du corps caverneux du pénis.

Il peut être avantageux, en particulier pour optimiser le confort, d'utiliser un bord arrière perpendiculaire (pour l'essentiel) à l'axe longitudinal.

Des rangées de reliefs sont formées dans le sens longitudinal, sur la face interne, avec un agencement en quinconce des reliefs pour réduire la taille des espaces entre les reliefs (la distance maximale inter reliefs pouvant être inférieure à 5 ou 7 mm, par exemple, sur la face interne incluant la portion de surface pour le contact scrotal, ou au moins pour ce qui concerne une partie formant moitié de la face interne). Plus généralement, il est formé un réseau de reliefs, avec des rangées d'au moins trois ou quatre reliefs suivant la direction longitudinale.

Selon une particularité, la face interne présente une conformation annulaire permettant de définir un passage traversant dont l'extension radiale mesurée perpendiculairement à l'axe longitudinal est au minimum de 27 mm et au maximum de 53 mm dans un état non déformé (matériau non étiré).

Le dispositif peut présenter une dimension longitudinale (mesurée suivant l'axe longitudinal) plus grande que l'épaisseur maximale du dispositif.

Selon une particularité, le dispositif présente une forme qui se distingue de la forme torique par une section oblongue plutôt que circulaire (section oblongue selon une coupe faite dans un plan parallèle à l'axe longitudinal).

A titre d'exemple non limitatif, le passage traversant délimité par la face interne est apte à être déformé par déformation élastique du matériau souple constitutif du dispositif, la distance entre les deux bords annulaires étant de préférence comprise entre 15 et 24 mm au moins du côté de la portion de surface pour le contact scrotal.

Optionnellement, les reliefs sont sous la forme de bossages, le dispositif comprenant un matériau polymérique souple pour définir les bossages (un réseau de bossages constitue un bon compromis entre l'exigence de confort et l'adhérence à la peau pénienne et/ou à la peau scrotale).

Selon une particularité, la dureté Shore A du matériau souple est avantageusement comprise entre 8 et 30, par exemple de l'ordre de 10 ou 15. Une dureté faible améliore significativement le confort car le matériau est plus doux, tandis que les bossages ou reliefs similaires améliorent l'effet antidérapant.

Lorsque les bossages sont sous forme de demi-billes, celles-ci peuvent présenter des diamètres différents, par exemple en fonction de leur position, afin d'améliorer l'adhérence et donner une forme interne générale plus arrondie.

Il peut être avantageux de former au moins 5 bossages par cm² de surface interne d'adhérence. Selon une option, il est prévu au moins 8 bossages par cm², cette disposition étant valable de préférence sur la totalité de la face interne. Ainsi, la dureté typiquement faible du matériau se combine avec l'irrégularité de la surface interne d'adhérence pour obtenir un très bon compromis entre le confort et le maintien en position.

Selon une particularité, le périmètre des bossages (et donc la taille de ces bossages) est croissant en s'éloignant des deux bords annulaires du dispositif.

Un dispositif selon l'invention peut comporter l'une ou plusieurs des caractéristiques suivantes :
- la portion de surface pour le contact scrotal correspond à une surface d'au moins 2 cm² et présente des premiers reliefs qui font saillie radialement vers l'intérieur (vers l'axe longitudinal).
- la portion de surface pour le contact scrotal, qui correspond de préférence à une surface d'au moins 3 cm², présente optionnellement au moins une zone de bordure dans laquelle des deuxièmes reliefs sont en saillie au moins en partie axialement vers l'extérieur.
- les deuxièmes reliefs sont répartis dans deux rangées qui forment tout ou partie des deux bords annulaires opposés, chacune des rangées s'étendant sur plus du quart de la circonférence du dispositif.
- les reliefs sont répartis en rangées parallèles.
- chaque rangée s'étend sur plus de la moitié de la circonférence interne du dispositif.
- il est prévu une zone de bordure arrière des reliefs et une zone de bordure avant des reliefs, chacune étant adjacente ou formant partie de l'un des bords annulaires du dispositif.
- les reliefs ont chacun une hauteur (mesurée perpendiculairement à l'axe longitudinal) d'au moins 0,4 mm.
- la face interne présente une conformation sensiblement circulaire ou au moins aussi haute que large.
- la portion de surface pour le contact scrotal présente, en section de coupe transversale, sensiblement le même rayon de courbure que celui de la portion de surface supérieure.
- la portion de surface pour le contact scrotal présente, en section de coupe transversale, un rayon de courbure supérieur au rayon de courbure de la portion de surface supérieure.
- la portion de surface pour le contact scrotal présente, en section de coupe transversale, un rayon de courbure inférieur au rayon de courbure de la portion de surface supérieure.
- le dispositif se présente sous la forme d'un anneau réalisé d'une seule pièce, en matière élastomère, de préférence un élastomère à base de silicone (bien entendu, un éventuel traitement de surface ne change en rien le fait que le dispositif provient d'une réalisation en une pièce).
- les moyens de retenue formant une fente font partie d'une pièce annulaire constituant le dispositif.
- les moyens de retenue délimitent un logement, distinct du volume intérieur, qui est formé intérieurement dans une partie inférieure du dispositif afin recevoir la partie de peau scrotale, le logement étant délimité par deux faces en regard qui présentent des reliefs de retenue (par exemple des nervures ou reliefs analogues définis entre des rainures).
- la fente est formée transversalement à l'axe longitudinal en ayant un premier débouché dans la face interne et un deuxième débouché, opposé au premier débouché, dans la face externe.
- la fente ou espacement analogue s'étend transversalement sur une portion de la périphérie de l'anneau, en débouchant extérieurement et/ou intérieurement.
- la fente ou l'espacement s'étend, transversalement à l'axe longitudinal, de façon intermédiaire (de préférence à équidistance) entre les deux bords annulaires.
- le débouché interne de l'espacement ou fente présente une forme générale d'arc de cercle correspondant à un secteur angulaire compris entre 90° et 270° autour de l'axe longitudinal de l'anneau.
- l'espacement ou fente radiale peut être délimité entre deux faces nervurées, en vis-à-vis, qui appartiennent à deux tronçons parallèles de l'anneau, agencés typiquement à une même distance de l'axe longitudinal.
- les deux tronçons parallèles appartiennent à une même pièce formant l'anneau.
- la face interne (qui s'étend de façon annulaire autour de l'axe longitudinal) définit un diamètre interne mesuré perpendiculairement à l'axe longitudinal, la fente formée par les moyens de retenue s'étendant entre deux extrémités espacées entre elles d'une distance supérieure au diamètre interne.
- le dispositif inclut une structure à au moins deux couches dans une partie inférieure du dispositif, les reliefs de la face interne étant au moins en partie formés par une couche dite intérieure définissant un revêtement, la fente étant formée entre deux couches en s'étendant sous la couche intérieure.
- la fente de l'anneau forme un espace d'expansion radiale entre deux couches, l'espace d'expansion radiale s'étendant le long d'une portion de circonférence déterminée plus petite que la circonférence intérieure (complète) de l'anneau.
- la fente s'étend de l'un à l'autre entre les deux bords annulaires de l'anneau.
- la circonférence déterminée ne dépasse pas deux tiers ou trois quarts de la circonférence intérieure de l'anneau, et est de préférence supérieure à un tiers de cette circonférence intérieure (plus généralement, la fente est formée sur plus d'un quart de tour et moins de trois quarts de tour autour de l'axe longitudinal de l'anneau).
- la fente, formant l'espace d'expansion radiale, peut être délimitée entre deux faces nervurées, en vis-à-vis, dont l'une appartient à un premier sous-élément d'anneau formant une couche proximale de l'axe longitudinal et l'autre appartient à un deuxième sous-élément d'anneau formant une couche distale de l'axe longitudinal (les premier et deuxième sous-éléments d'anneau étant concentriques).
- les deux sous-éléments appartiennent à une même pièce formant l'anneau.
- il est prévu une structure à au moins deux couches pour former le dispositif, les reliefs de la face interne étant au moins en partie formés par une couche de revêtement.
- deux couches de la structure sont définies par : une couche de base, ayant une épaisseur supérieure à un premier seuil sans dépasser un second seuil égal à 10 mm ; une couche de revêtement formant la face interne et dont l'épaisseur maximale est inférieure ou égale au premier seuil.
- le revêtement est rapporté/surmoulé sur la couche de base.
- les reliefs internes sont issus du moulage de la pièce formant l'anneau ; un traitement de surface ultérieur peut être réalisé pour améliorer l'adhérence par un traitement mat (sans modifier le profil des reliefs).
- les bossages forment des demi-billes, optionnellement réparties en au moins deux groupes de taille différente, qui recouvrent de préférence la totalité de la face interne de l'anneau ; ceci améliore la retenue/stabilisation de la peau scrotale et pénienne.
- la face interne présente, du côté de la portion de surface supérieure, l'un au moins parmi : une extension axiale décalée axialement vers l'avant par rapport au reste de la face interne ; et deux projections espacées entre elles d'au moins 5 mm et en regard l'une de l'autre, de part et d'autre d'un plan médian virtuel du dispositif, les deux projections étant radialement saillantes vers l'intérieur.

Selon une particularité, les deux projections forment localement un même surplus d'épaisseur du dispositif, qui est un maximum d'épaisseur du dispositif au moins du côté supérieur du dispositif.

Selon une particularité, les reliefs présentent un creux et/ou sont en forme de ventouses. Au moins pour une partie des reliefs, ceux-ci peuvent être plus larges que hauts, tout en ayant typiquement une hauteur de l'ordre de 1 mm (chaque relief étant significatif, parfaitement visible individuellement à l'œil nu).

Plus généralement de tels reliefs, suffisamment marqués, forment des espaces interstitiels importants qui favorisent la sudation.

Dans une forme de réalisation optionnelle, le corps de l'anneau présente une échancrure pour le passage de la peau scrotale, du côté du bord avant.

Selon une particularité, le plan médian séparant le dispositif en deux moitiés est un plan de symétrie, de préférence un plan de symétrie vertical coupant/traversant la portion de surface supérieure et la surface interne d'adhérence.

Optionnellement, les deux projections forment des points de contention, en servant à stabiliser et empêcher le glissement des testicules (glissement susceptible d'entraîner leurs descentes dans le scrotum). Ils améliorent la tenue des testicules en position supra-scrotale par l'action du corps formant l'anneau.

L'anneau, en enserrant le pénis et le scrotum, exerce une contre-poussée vers le haut permettant de maintenir les testicules en position voulue. Même pour ce qui concerne la partie du scrotum qui de fait ne peut être introduite dans l'anneau (partie de la zone périnée), on constate que celle-ci est comme tendue et maintenue vide par la contre pression exercée par l'anneau enserrant le pénis et le scrotum.

L'option avec les deux projections (formant des points de contention) limite avantageusement le risque que les testicules puissent tout de même migrer vers la zone du périnée (anneau mal mis, pratique sportive, etc.), entrainant un léger glissement de l'anneau sur le pénis et le scrotum et donc relâchant la contre pression exercée. En effet, les projections contribuent à barrer la route aux testicules en exerçant une pression sur les voies de descentes autour de l'anneau vers la zone du périnée.

Optionnellement, le matériau constitutif du dispositif est imperméable, de préférence hydrophobe. Ce caractère hydrophobe permet typiquement de définir un angle de contact avec l'eau liquide (eau sous forme de gouttelette/goutte) supérieur à 100°. Cet angle est par exemple de l'ordre de 110-111 ° pour un matériau de type silicone.

Selon une particularité, le corps en matériau souple formant l'anneau est plein, en étant dépourvu de cavités internes entre la face interne et la face externe.

A titre d'exemple non limitatif, l'épaisseur moyenne du matériau dans le corps formant l'anneau peut être comprise entre 1,5 et 7 mm, de préférence entre 2 et 5 mm. Ainsi, on minimise l'encombrement externe du dispositif autour de la verge.

La résistance à la traction du matériau peut être d'au moins 5 MPa.
Par ailleurs il est permis une utilisation du dispositif afin de placer aisément, maintenir et assurer l'efficacité de l'effet contraceptif par déplacement des testicules.

Plus particulièrement, on utilise le dispositif pour permettre une contraception masculine, en encerclant une verge et la peau scrotale du scrotum vide, en particulier pendant une durée quotidienne supérieure ou égale à 14 ou 15 heures, de façon à ce que les reliefs de la face interne soient en prise avec la peau scrotale et exercent une pression vers le haut ou anti-gravitaire, ce qui maintient les testicules dans une position proximale par rapport au canal inguinal (c'est-à-dire en position supra-scrotale, au niveau de la base du pénis).

L'effet de remontée ou effet antigravitaire du dispositif, fonctionnant comme une exoprothèse, empêche les testicules de s'éloigner des zones chaudes du corps (ce qui induit un réchauffement de ces testicules par comparaison avec une position plus basse).

Selon une particularité, des zones interstitielles s'étendent entre les reliefs (ce qui peut grandement faciliter la sudation), de sorte que le dispositif est particulièrement adapté pour un port prolongé, de préférence pour une durée de plus de douze heures.

L'installation est simple : elle peut se faire assis, debout, couché sur le dos, couché sur le côté. Le maintien des testicules est même décrit comme agréable et plaisant pour de nombreux usagers.

Une option d'installation du dispositif peut consister, pour l'utilisateur, à se coucher sur le dos et faire glisser le dispositif autour de la verge, suivant une direction égale ou similaire à la direction de la gravité, puis remonter la peau scrotale (scrotum vide) dans le volume intérieur formé par le corps annulaire du dispositif et/ou dans une fente des moyens de retenue. Que ce soit en position debout ou en position assise, le dispositif enserre et soutient par le bas la peau scrotale insérée dans le volume intérieur ou dans fente, respectivement, grâce à la portion de surface pour le contact scrotal (portion de surface qui fait partie de la surface interne d'adhérence ou qui délimite la fente).

Un surplus local d'épaisseur du dispositif dans cette zone peut faciliter l'effet de soutien du scrotum vide.

Typiquement, l'efficacité de l'effet contraceptif est vérifiée par des analyses de sperme régulières, par exemple tous les 3 mois, appelées spermogrammes. Avant le port de l'anneau, il peut être opportun de faire une analyse pour vérifier la fertilité de la personne.

On prévoit une durée de port de l'anneau de l'ordre de 15h par jour, sans interruption. Cette durée est indicative, sachant qu'une journée exceptionnelle avec une durée de port inférieure n'est pas problématique.

Le diamètre interne du dispositif peut être choisi parmi plusieurs tailles, par exemple trois tailles, ou au moins cinq ou six tailles. On peut retrouver les catégories habituelles « S » pour un diamètre faible qui ne dépasse pas 30 ou 33 mm, « M » pour un diamètre moyen (de l'ordre de 35 ou 40 mm) et « L » pour un grand diamètre de l'ordre de 43 ou 45 mm. Une catégorie « XL » pour une taille très large peut par exemple correspondre à un diamètre de 50 mm environ. Une catégorie « XXL » peut aussi être prévue, par exemple avec un diamètre interne de 52 ou 53 mm. De manière analogue, une catégorie « XS » pour une taille petite peut par exemple correspondre à un diamètre de 25 ou 26 mm environ. Une catégorie « XXS » peut aussi être prévue, par exemple avec un diamètre interne de 23 mm environ. En tout état de cause, le diamètre interne sera typiquement compris entre 20 et 55 mm, de préférence entre 23 et 52 mm.

Du fait du choix du diamètre le plus approprié, le port de ce dispositif est aisément tolérable. Le dispositif de contraception masculine peut être compact et pourvu de points de serrage ou contention, de sorte à être compatible avec un grand nombre d'activités, y compris les plus physiques ou qui demandent les mouvements les plus inhabituels. Concernant la position couchée sur le ventre qui peut être moins agréable, celle-ci reste inhabituelle et n'est pas prise longtemps pour la période d'éveil de l'utilisateur.

Il est également proposé un procédé de fabrication du dispositif de contraception masculine selon l'invention, par utilisation d'un moule, le procédé comprenant les étapes consistant essentiellement à :
- fournir un matériau polymère réticulé/thermodurcissable, de préférence à base de silicone,
- mouler en forme d'anneau, à chaud, le matériau polymère thermodurcissable dans une cavité annulaire du moule, en formant les reliefs de la face interne par moulage dans des empreintes creuses correspondantes du moule situées en regard d'une partie périphérique externe du moule, et
- refroidir ledit matériau pour permettre son durcissement et obtenir un état solide et souple du dispositif.

Le polymère durcissable peut être du type hautement étirable, par exemple apte à s'allonger avant la rupture de plus de 400 ou 500 %, ce qui permet de facilement obtenir une conformation étirée très ovalisée du dispositif. Typiquement, l'allongement peut être choisi dans la gamme 600-1000 %(norme DIN 53 504 S2). Cela limite fortement le risque de striction en portant périodiquement le dispositif, par exemple 15 heures par jour environ.

Le dispositif peut être optionnellement obtenu par moulage par injection d'un mélange prêt à l'emploi (de composants A et B). De façon connue en soi, ces composants sont directement introduits dans la machine de moulage par injection depuis les récipients initiaux au moyen d'une unité de dosage et de mixage. Le mélange, composé des deux composants dans le rapport 1: 1, est injecté dans le moule chauffé. A titre d'exemple, la puissance de chauffe correspond à des températures de moule de 170 - 230 °C, le caoutchouc de silicone réticulé par addition se vulcanise en quelques secondes, sans produits de dissociation.

La haute vitesse de durcissement et le démoulage facile permettent une production entièrement automatisée d'un grand nombre d'articles dans des temps de cycle brefs.

A titre d'exemple, on peut utiliser un matériau résultant d'un mélange de deux composants élastomères ou silicones (optionnellement ayant chacun une viscosité similaire, par exemple de l'ordre de 70 au sens de la norme DIN 53018 à 20°C). Le mélange vulcanisé/réticulé peut présenter une densité de 1,07 g/cm³ et une dureté de l'ordre de 8 (dureté Shore A).

Le dispositif peut être conçu à base de Silopren LSR 2010 TP 3740, le matériau étant ainsi un caoutchouc de silicone standard obtenu par moulage par injection bi-composant.

Plus généralement, le matériau peut être à base de silicone et présente une résistance à la traction comprise entre 1,9 et 5 N/mm² (selon la norme de mesure DIN 53 504 S2).

Optionnellement, la résistance au déchirement peut être comprise entre 3 et 13 N/mm, de préférence entre 4 et 9 N/mm.

D'autres caractéristiques et avantages de l'invention apparaîtront au cours de la description suivante de plusieurs de ses modes de réalisation, donnés à titre d'exemples non limitatifs, en regard des dessins joints dans lesquels :
- la figure 1 est une vue en perspective d'un dispositif de contraception masculine conforme à un premier mode de réalisation de l'invention ;
- la figure 2 est une vue de côté d'un dispositif de contraception masculine conforme à un deuxième mode de réalisation de l'invention;
- la figure 3 est une vue en perspective du dispositif de la figure 2 ;
- la figure 4 est une vue en perspective du dessous d'un dispositif selon un troisième mode de réalisation similaire à celui de la figure 3 ;
- la figure 5 est une vue de face d'un dispositif de contraception masculine dont les faces avant et les faces arrière sont identiques, selon un quatrième mode de réalisation ;
- la figure 6A est une vue d'une moitié d'un dispositif de contraception masculine dans une option avec une partie inférieure inclinée de l'avant vers l'arrière, ce qui permet de former une section de passage plus grande du côté du bord arrière par rapport à la section de passage délimitée par le bord avant;
- la figure 6B est une vue d'une moitié d'un dispositif similaire à celui de la figure 2 permettant d'illustrer des sections de coupe dans le corps annulaire du dispositif ;
- la figure 7 est une vue de côté montrant un dispositif de contraception masculine qui se différencie notamment du deuxième mode de réalisation par une échancrure du côté de la face avant ;
- la figure 8A montre un dispositif de contraception masculine conforme à un autre mode de réalisation de l'invention, avec une fente séparant deux sous-éléments de la partie inférieure du dispositif ;
- la figure 8B est une vue, suivant une coupe médiane verticale, d'une moitié du dispositif de la figure 8A ;
- la figure 9A est une vue en perspective d'un dispositif de contraception masculine conforme à un autre mode de réalisation de l'invention, avec un espace séparant deux couche de la partie inférieure du dispositif ;
- la figure 9B est une vue, suivant une coupe médiane horizontale, d'une partie supérieure du dispositif de la figure 9A ;
- la figure 10 est une vue en coupe verticale montrant un détail du dispositif des figures 9A et 9B.
Sur les figures, les mêmes références désignent des éléments identiques ou similaires.

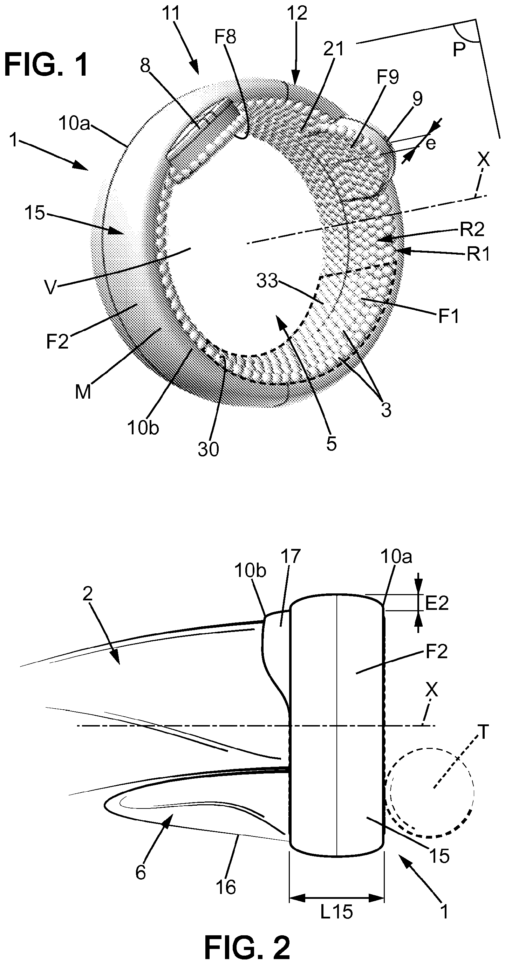

En référence aux figures 1, 2 et 3, le dispositif de contraception masculine 1 présente une forme générale annulaire permettant d'encercler la verge 2 du porteur. La face interne F1 du dispositif 1 présente des irrégularités résultant de reliefs 3. Une surface interne d'adhérence 30, typiquement continue, peut être constituée avec de tels reliefs 3. Dans cet exemple non limitatif, le dispositif 1 s'étend de façon continue autour d'un axe longitudinal virtuel X. Un tel axe X peut optionnellement appartenir à un plan médian de symétrie P qui sépare le dispositif 1 en deux moitiés 11, 12.

La face externe F2 du dispositif 1 peut s'étendre entre deux extrémités axiales opposées. La face externe F2 est par exemple lisse et forme une circonférence continûment arrondie. Ici, les deux extrémités axiales peuvent être définies par deux bords annulaires respectifs 10a, 10b. Le bord arrière 10a est conçu et agencé pour venir buter du côté de la racine de la verge 2 à l'état monté du dispositif 1 avec la verge 2 encerclée.

Comme illustré à la figure 2, le dispositif de contraception masculine 1 permet d'encercler la verge 2 et le scrotum vide 6 de l'individu porteur en âge de de procréer. Un bord annulaire arrière 10a, qui peut s'étendre transversalement à l'axe longitudinal X (disposition perpendiculaire dans le cas de la figure 2) du dispositif 1 vient buter du côté de la racine de la verge. La face interne F1, qui délimite un volume intérieur V du dispositif 1, présente des reliefs 3 régulièrement répartis ou resserrés entre eux au moins au niveau de la surface interne d'adhérence 30.

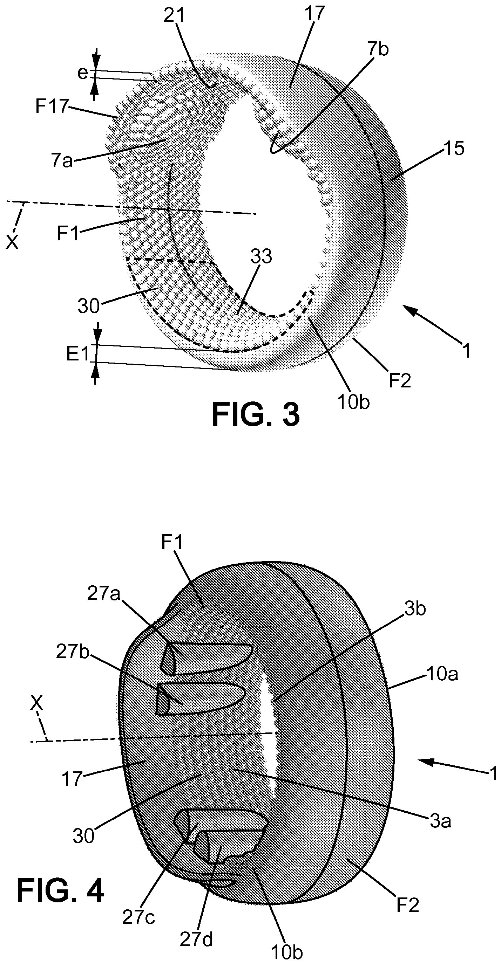

Le dessus de la verge 2 peut s'engager contre une portion de surface supérieure 21, concave, du dispositif 1, visible sur les figures 1 et 3. A l'opposé de cette portion de surface supérieure 21, la surface interne d'adhérence 30 présente une portion de surface antidérapante 33.

Dans l'exemple des figures 1 à 7, cette portion de surface antidérapante 33 sert pour le contact scrotal, et est en pratique espacée de la verge 2 afin de permettre le maintien d'une partie de peau scrotale dans une zone inférieure du volume intérieur V avec en contact direct contre les reliefs 3. Comme l'espacement entre la verge 2 et les reliefs 3 en regard est suffisamment petit, le scrotum vide 6 est maintenu serré dans le volume intérieur V sans que les testicules T puissent pénétrer dans ce volume V. En effet, le bord annulaire arrière 10a repousse les testicules T en arrière du volume intérieur V.

La portion de surface 33 pour le contact scrotal, définie à l'aplomb de la portion de surface supérieure 21 pour assurer une pression sur le scrotum vide 6, correspond à une surface d'au moins 2 ou 3 cm².

Dans les formes de réalisation illustrées, la surface interne d'adhérence 30 s'étend sur la majorité ou la totalité de la face interne F1, de sorte que des reliefs 3 sont présents dans des zones opposées l'une de l'autre.

Ici, la portion de surface supérieure 21, concave, de la face interne F1 permet le contact avec la verge 2 du côté de sa veine dorsale superficielle en procurant un effet de guidage et de stabilisation. Des reliefs 3, ici sous la forme de bossages, peuvent être prévus dans cette portion de surface supérieur 21. La face interne F1 peut être localement allongée (de préférence vers l'avant) et/ou pourvue de points d'appui ou contention, ce qui permet d'augmenter la zone de contact entre la verge 2 et cette portion supérieure 21 de surface.

La formation de reliefs 3, typiquement des bossages agencés en rangées parallèles R1, R2, peut permettre d'obtenir un effet antidérapant en combinaison avec une dureté relativement faible du matériau M constitutif du dispositif 1 (par exemple une dureté inférieure ou égale à 30 selon l'échelle Shore A). Cet effet antidérapant peut être fourni au moins dans la portion de surface supérieure 21, afin de limiter le risque de glissement du dispositif 1 lors de phases de déplacement du porteur (marche, course, saut, etc.).

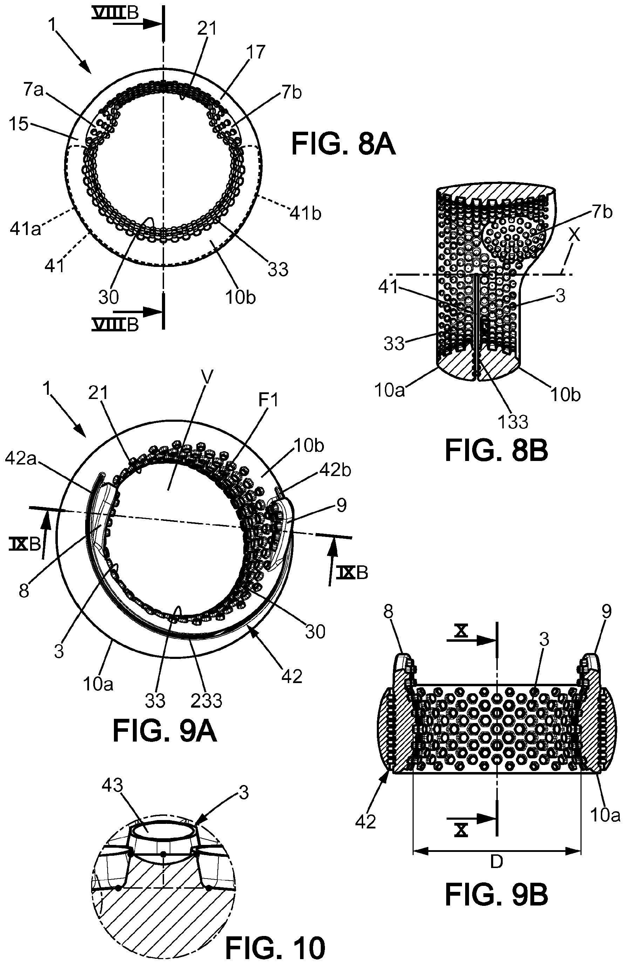

Dans les options illustrées sur les figures 1 à 7, le dispositif 1 peut également comporter le même type de reliefs 3 pour former la surface interne d'adhérence 30 et la portion de surface supérieure 21. Alternativement, on peut prévoir au moins deux groupes de reliefs structurellement différents. A titre d'exemple non limitatif, on peut prévoir une rugosité résultant d'aspérités submillimétriques dans une zone, tandis que l'autre zone présente des bossages ayant une extension d'au moins 1 mm. Dans d'autres variantes, comme celles illustrées sur les figures 8A à 9B, un groupe de reliefs est formé intérieurement dans une fente 41 ou 42 pour former une zone d'adhérence qui prolonge ou qui s'étend parallèlement à la surface interne d'adhérence 30.

De préférence, on forme plusieurs dizaines de bossages, visibles chacun à l'œil nu, pour constituer la surface d'adhérence 30. Dans les exemples des figures, les reliefs 3 sont typiquement des bossages agencés en rangées parallèles qui entourent chacune entièrement l'axe longitudinal X. Comme bien visible sur les figures 1 et 3, ces reliefs 3 sont ici répartis de l'un à l'autre des bords 10a, 10b au moins dans la partie inférieure du dispositif destinée au contact avec le scrotum vide 6.

Comme illustré sur la figure 1, le volume intérieur V qui est délimité par la face interne F1 peut correspondre à un volume de contour sensiblement cylindrique ou continûment arrondi, dans un état non déformé du dispositif 1. L'extension longitudinale de ce volume intérieur V est limitée et typiquement inférieure à la plus grande largeur ou diamètre interne D définie par le passage traversant 5 (passage central entouré par la face interne F1). Ce volume intérieur V présente une largeur typiquement inférieure ou égale à 50 ou 53 mm mais supérieure à 27 mm, de sorte qu'il peut être rempli par un tronçon de la verge 2 et par le scrotum vide 6.

Pour faciliter l'insertion de la verge 2 au travers du passage traversant central 5, la face interne F1 peut présenter une circonférence généralement circulaire avec de possibles exceptions, notamment lorsqu'il est prévu une ou plusieurs projections 7a, 7b comme dans le cas illustré sur la figure 3.

Les projections 7a, 7b font par exemple saillie vers l'intérieur (par rapport au reste de la face interne F1) d'une hauteur d'au moins 1 ou 2 mm et/ou, typiquement, peuvent avoir une dimension d'extension vers l'axe longitudinal X qui est supérieure à la hauteur des reliefs 3. Ces projections 7a, 7b peuvent optionnellement inclure, chacune, des reliefs 3 sous forme de bossages, comme dans le reste de la face interne F1.

Les projections 7a, 7b limitent l'un des types de descente des testicules T. Le chemin d'éventuel contournement de l'anneau est en effet bloqué par les projections 7a, 7b empêchant les testicules T de migrer en zone périnée. Les projections 7a, 7b sont souples, typiquement avec une capacité de déformation importante pour s'adapter à la morphologie de chacun et aux différents mouvements et postures.

Dans l'exemple non limitatif de la figure 3, l'espacement entre les deux projections 7a, 7b, par exemple compris entre 14 et 40 mm, est tel qu'il est formé une zone de gorge dans une portion supérieure 21 de la surface interne d'adhérence 30. Optionnellement, ces deux projections 7a, 7b peuvent former des points de contention qui sont adjacents au bord arrière 10 (du côté de la racine de la verge) et se situent :
- de part et d'autre du plan médian P, qui est éventuellement un plan de symétrie ;
- l'un dans un secteur angulaire qui peut être compris entre 22° et 67° et l'autre dans un autre secteur angulaire qui peut être compris entre 292° et 337° (sachant que la référence 0/360° correspond au point supérieur de la face interne F1 du dispositif 1, c'est-à-dire le centre de la portion de surface supérieure 21 en regard de la veine dorsale superficielle du pénis.

En référence à présent à la figure 4, on peut voir un nombre de projections supérieur à deux. Ici, quatre organes saillants 27a, 27b, 27c, 27d, sont formés sur la face interne F1, avec chacun une zone amincie et/ou fuselée pour définir un bord de fuite orienté vers l'arrière et éventuellement placé à distance du bord annulaire arrière. Pour obtenir un effet de stabilisation, la surface dans ces organes saillants 27a, 27b, 27c, 27d peut être rugueuse. Ces organes saillants peuvent être réunis et rapprochés par paires pour être localisés, dans la face interne F1, de façon similaire à la localisation précitée dans le cas des deux projections 7a, 7b.

Que ce soit avec le dispositif de la figure 3 ou celui de la figure 4, l'espacement en forme de gorge (entre les projection/organes saillants) dans la portion de surface supérieure 21 peut contribuer à permettre de déformer le dispositif en le rendant légèrement moins courbé du côté de la portion de surface supérieure 21. Cette déformation peut permettre de mieux répartir l'effort de retenue du côté inférieur, en enserrant aussi latéralement la peau scrotale 16 (ce qui résulte d'un léger effet de déformation en trapèze inversé). Autrement dit, l'écartement des points de contention peut correspondre à une position optimale de soutien des testicules sans opérer de compression du pénis.

Le matériau constitutif du dispositif 1 est peu compressible ou incompressible et de densité au moins égale à 1, par exemple au moins égale à 1,04. C'est un matériau souple M qui présente une extensibilité importante. Lorsque le dispositif 1 est réalisé d'une pièce mono-matière, ce matériau M peut présenter des propriétés antidérapantes. Il s'agit typiquement d'un matériau silicone.

Bien entendu, le matériau souple M du dispositif 1 peut être étiré sans risque de déchirure, en particulier dans des directions radiales, sachant que son allongement avant la rupture est au moins de 400 % par exemple, de préférence de 650 à 1000%.

La figure 1 montre une option dans laquelle le dispositif 1 comprend une paire d'éléments saillants 8, 9 faisant saillie, vers le côté avant, par rapport au corps 15 en forme d'anneau régulier/d'extension axiale constante. Ces éléments saillants 8, 9 dépassent par exemple au maximum de 5 mm vers l'avant par rapport au reste du bord avant 10b. Ici chacun des éléments saillants 8, 9 présente une portion frontale dont le profil est en "C" en formant ainsi une face frontale continûment convexe de l'une à l'autre des jonctions avec la partie circulaire du bord avant 10b. D'autres profils, formant par exemple des vagues, peuvent aussi être utilisés. Les éléments saillants 8, 9 définissent des extensions axiales F8, F9 de la face interne F1 permettant de stabiliser davantage le dispositif 1.

Les reliefs 3 peuvent être présents du côté intérieur de ces éléments saillants 8, 9 mais pas sur la face frontale à l'exception éventuelle d'une rangée ou bordure de reliefs 3b saillants vers l'avant depuis la face frontale définie dans l'élément correspondant 8 ou 9.

L'épaisseur e des éléments saillants 8, 9 ou de toute autre extension 17 analogue peut être inférieure à l'épaisseur E2 du corps 15 dans la partie supérieure, par exemple en étant deux fois plus petite que cette épaisseur E2. En référence à la figure 3, l'extension axiale F17 qui prolonge vers l'avant la face interne F1, du côté supérieur, est concave en suivant la courbure générale du bord avant 10b. Le rayon de courbure de l'élément saillant 17 peut être ainsi le même que dans le corps 15 principal du dispositif 1.

On comprend que les éléments saillants 8, 9 et les reliefs 3, répartis ici dans plusieurs zones en regard dans la face interne F1, améliorent la tenue générale du dispositif 1 à la racine de la verge 2. Les reliefs 3 peuvent être directement intégrés au matériau souple M constitutif du dispositif. Dans des variantes, le matériau souple M n'est pas l'unique composant du dispositif 1 et peut seulement former un revêtement rapporté/sourmoulé sur une autre couche de matériau souple et étirable.

Par ailleurs, comme visible sur la figure 9A, il peut être prévu de former, par une fente 42, un espace d'expansion radiale qui s'étend entre deux sous-éléments formant chacune une couche respective de l'anneau. La couche proximale par rapport à l'axe longitudinal X peut ainsi présenter une déformation élastique accrue, ce qui offre un surplus de confort pour l'utilisateur. La fente 42 pour définir un tel espace peut être obtenue lors du moulage du corps en silicone. Une telle fente 42 peut être prévue dans une large gamme de dispositifs conformes à l'invention. Alternativement, on peut prévoir une fente 41 qui s'étend en travers du corps du dispositif 1, pour l'essentiel dans un plan perpendiculaire à l'axe longitudinal A comme dans le cas illustré sur les figures 8A et 8B. Cette conception peut aussi permettre une expansion avec effet d'écartement entre les bords 10a et 10b.

Les figures 2 et 3 montrent une option avec un élément saillant unique 17 plus large que les éléments 8, 9 et qui est réparti dans les deux moitiés 11 et 12. Ce type d'éléments saillants 8, 9, 17 peut être formé du côté du dessus du dispositif 1, afin de coopérer avec le dessus de la verge 2, de part et d'autre de la veine dorsale superficielle. Lorsque de tels éléments saillants 8, 9, 17 sont présents, les reliefs 3 de la face interne F1 peuvent aussi être prévus sur le côté intérieur de ces éléments saillants 8, 9, 17.

La présence d'éléments saillants 8, 9, 17 permet d'allonger localement la surface interne en prise avec la verge 2. Plus généralement, on comprend que la face interne F1 peut présenter, de préférence du côté de la portion de surface supérieure 21, l'un au moins parmi :
- une extension axiale (résultant d'un élément saillant 8, 9 ou 17) décalée axialement vers l'avant par rapport au reste de la face interne F1 ; et
- deux projections 7a, 7b espacées entre elles et en regard l'une de l'autre de part et d'autre du plan médian virtuel P du dispositif 1, les deux projections 7a, 7b étant radialement saillantes vers l'intérieur.

Dans une partie du dispositif 1 complémentaire de celle qui comprend l'extension 17 ou les éléments 8, 9, il peut être prévu de former un espacement ou une fente 41, 42 qui s'étend transversalement sur une portion de la périphérie de l'anneau, comme illustré par exemple sur les figures 8A et 9A.

Dans une variante, le dispositif 1 peut consister en un anneau présentant une symétrie de révolution autour de l'axe longitudinal X. Dans ce cas, le dispositif 1 peut être constitué uniquement par le corps 15 montré sur la figure 1, sans les éléments saillants 8, 9.

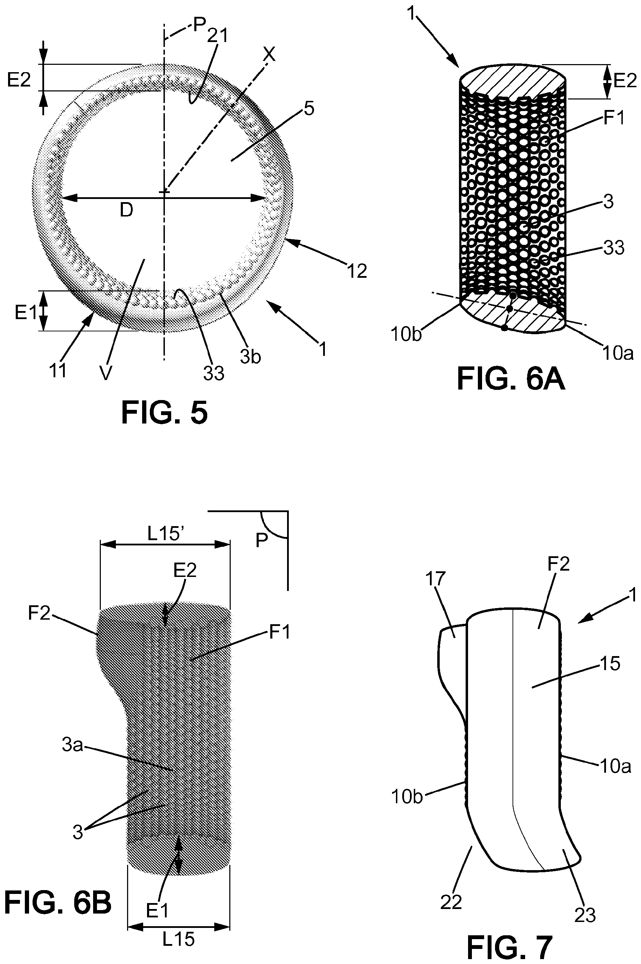

Par ailleurs, comme illustré sur les figures 5 et 6B, la face externe F2 et la face interne F1 peuvent présenter entre elles une épaisseur qui varie. L'épaisseur E1 mesurée dans la partie inférieure du dispositif 1, ici dans le plan médian P, peut être supérieure, par exemple supérieure d'au moins 1 mm, à l'épaisseur E2 mesurée dans la partie supérieure du dispositif 1, ici dans le plan médian P.

Optionnellement, le dispositif 1 se décompose en deux moitiés identiques 11, 12, séparées entre elles par le plan médian P, et peut en outre être dépourvu de portions saillantes vers l'avant ou vers l'arrière. Eventuellement, le dispositif 1 peut présenter une face avant et une face arrière qui sont identiques. Cela permet de mettre en place le dispositif 1 sur la verge 2 sans risque de se tromper de sens d'insertion.

Dans des modes de réalisation, les reliefs 3 de la face interne F1 peuvent se présenter sous la forme de bossages, par exemple sous la forme de demi-billes intégralement formées avec la couche formant la face interne F1. Bien que les figures montrent une face interne F1 continûment pourvue de reliefs 3, on peut aussi prévoir des zones sans reliefs.

On peut prévoir au moins deux séries de reliefs, structurellement différents ou non. Dans l'exemple de la figure 1, les reliefs 3b en bordure se différencient des autres reliefs 3a en ce qu'ils font aussi saillie vers l'avant ou vers l'arrière par rapport à la face externe F2, respectivement selon qu'il se situent le long du bord arrière 10a ou du bord avant 10b.

Les demi-billes ou bossages similaires recouvrant l'intérieur du corps 15 en forme d'anneau améliorent la retenue/stabilisation de la peau scrotale 16 et de la peau pénienne. Ils permettent d'améliorer le maintien des testicules T en position supra-scrotale à la racine de la verge à proximité de l'orifice externe du canal inguinal pour induire une élévation de la température des testicules avec le corps humain comme seule source de chaleur.

Sur la figure 1, on a représenté des reliefs 3 agencés dans des rangées respectives R1, R2 et qui sont, selon une option, répartis en quinconce. Avec ce type de structure pour former la face interne F1, il est intéressant de définir au moins trois ou quatre rangées parallèles de reliefs. Dans cet exemple non limitatif, chaque rangée R1, R2 est disposée de façon généralement parallèle aux bords 10a et 10b.

L'extension axiale L15 du corps 15 est comprise entre 15 et 24 mm et peut être constante (cf. distance mesurée longitudinalement entre les deux bords 10a, 10b) hormis dans la partie supérieure où sont prévus les éléments saillants 8, 9, 17 optionnels (avec une extension axiale L15' localement plus grande). Lorsque l'extension axiale L15 est supérieure à 15 mm, on peut prévoir dans la face interne F1 au moins sept ou huit rangées différentes ou tout du moins sept ou huit reliefs ou davantage placés sur un tronçon longitudinal allant du bord arrière 10a au bord avant 10b.

En référence aux figures 5 et 6A-6B, on peut voir que la face interne F1 du corps 15 peut présenter une largeur constante (correspondant à l'extension axiale/longitudinale L15, et typiquement choisie entre 15 et 24 mm) mais moins d'épaisseur du côté supérieur du dispositif 1. L'épaisseur réduite E2 permet de favoriser la déformation et le confort de port. La dureté shore A du matériau constituant la face interne est comprise entre 8 et 30, de préférence entre 10 et 20.

Ce type de structure et le contact doux rend le dispositif 1 particulièrement bien adapté pour la pratique de sport régulière ou intensive. La présence additionnelle de projections 7a, 7b comme celles visibles sur la figure 3 est également compatible avec la pratique du sport.

Dans les options avec une symétrie par rapport au plan P, l'axe longitudinal X virtuel constitue un axe central équidistant entre la portion de surface supérieure 21 et la portion de surface 33. Optionnellement, comme visible sur la figure 6A, on peut prévoir un élargissement de l'ouverture d'accès, du côté du bord arrière 10a. Ici, le bord arrière 10a descend plus bas que le bord avant 10b en raison de l'inclinaison de la partie inférieure du corps formant le dispositif 1.

On peut aussi prévoir une épaisseur de l'anneau pénoscrotal qui augmente progressivement en s'éloignant de chacun des bords annulaires 10a, 10b (gradient d'épaisseur dans l'anneau), par exemple avec un maximum d'épaisseur sensiblement à mi-distance entre les deux extrémités annulaires axiales. L'épaisseur E1 peut être la même, avec un même profil, ce qui est intéressant pour former un anneau qui se porte dans les deux sens, avec les bords 10a, 10b interchangeables pour former l'avant et l'arrière.

Par ailleurs, les reliefs 3 du dispositif 1 peuvent présenter une plus grande section en s'éloignant des deux bords 10a, 10b. Cette augmentation de section rend le dispositif 1 confortable même avec une hauteur importante des reliefs 3, par exemple supérieure ou égale à 0,9 ou 1 mm. Les zones interstitielles entre les bossages ou reliefs 3 peuvent faciliter la sudation, ce qui rend le dispositif 1 particulièrement adapté pour un port prolongé, typiquement pour une durée de plus de douze heures.

Par rapport au dispositif de la figure 5, les dispositifs des figures 1, 2-3, 4 et 7 présentent l'avantage de mieux répartir la pression sur le dessus de la verge 2, grâce aux éléments 8, 9, 17 saillants dans la direction avant. Le fait d'étendre la face interne F1, avec de telles extensions axiales, complète le caractère déformable et procure un avantage de confort important pour les personnes passant de longues périodes assises durant le port du dispositif 1.

La dimension caractéristique du passage traversant 5, ici un diamètre D interne, peut être de l'ordre de 40 mm. Pour un port confortable du dispositif 1, il peut être intéressant pour l'utilisateur de choisir sa taille, par exemple parmi des diamètres D de 30, 40 ou 50 mm.

En référence à présent aux figures 8A et 8B, on peut voir que la partie inférieure du dispositif 1, plus épaisse, peut être optionnellement traversée par une fente 41.

Indépendamment de la façon de réaliser les reliefs 3 et de former la partie supérieure, la fente 41 peut présenter un intérêt en ce qu'elle forme un passage permettant d'insérer une extrémité de la poche scrotale vide (partie de peau scrotale) avec un effet de retenue, assez comparable à la retenue d'une sangle par exemple. L'élasticité du matériau du dispositif 1 permet d'écarter cette fente 41 au moment de l'insertion. Si cette extrémité est glissée au travers de la fente 41 pour ressortir en bas du côté de la face externe F2, la fente 41 écartée se referme ensuite et on minimise le risque que la partie de peau scrotale ressorte par l'arrière du volume intérieur V.

Bien entendu, avec ce dispositif 1 pourvu d'une fente 41, l'utilisateur peut choisir aussi bien de placer la partie de peau scrotale 16 dans la fente 41 ou dans une partie inférieure du volume intérieur V, comme dans le cas illustré dans la figure 2. Les extrémités 41a, 41b de la fente 41 sont éloignées l'une de l'autre, ici en étant diamétralement opposées, ce qui permet une adaptation à une large variété de peau scrotale 6.

Comme montré sur la figure 8B, les faces internes délimitant la fente 41 peuvent être nervurées, rainurées ou texturées pour améliorer l'adhérence contre la partie de peau scrotale 16, pour une meilleure retenue. Plus généralement, on peut prévoir n'importe quels moyens de retenue pour former la fente 41 et définir une ou plusieurs portions de surface 133 contre laquelle la peau scrotale 6 est engagée avec une forte adhérence.

En référence à la figure 9A, on peut voir que les éléments saillants 8, 9 peuvent être placés à mi-hauteur du dispositif 1, ces éléments saillants étant optionnels et interchangeables avec l'extension 17 ou extension similaire comme montré sur la figure 8B. Par ailleurs, il peut être prévu une fente 42 définissant un espace d'expansion radiale dans la partie inférieure du dispositif 1, ici sous la forme d'une entaille allant du bord avant 10b au bord arrière 10a. Ce type d'entaille permet une mobilité de la partie inférieure du dispositif 1 avec un effet de dilatation dans une direction radiale.

Ce type de déplacement radial peut accompagner une dilatation naturelle du scrotum.

La peau scrotale 6 peut éventuellement être glissée sélectivement au travers de cette fente 42, depuis le bord arrière 10a, pour ressortir du côté du bord avant 10b. La fente 42 qui est écartée pendant cette insertion de la peau scrotale 6 peut se refermer ensuite et on minimise le risque que la partie de peau scrotale 16 ressorte par l'arrière du volume intérieur V.

Plus généralement, on peut prévoir n'importe quels moyens de retenue pour former la fente 42 et définir une ou plusieurs portions de surface 233 contre laquelle la peau scrotale 6 est engagée, avec une forte adhérence.

Bien entendu, avec ce dispositif 1 pourvu d'une fente 42, l'utilisateur peut choisir aussi bien de placer la partie de peau scrotale 16 dans la fente 42 ou dans une partie inférieure du volume intérieur V, comme dans le cas illustré dans la figure 2.

Comme illustré sur la figure 9B, les faces internes délimitant l'espace 42 peuvent être nervurées ou texturées pour améliorer l'adhérence contre la peau scrotale 6, pour une meilleure retenue. Dans l'exemple illustré, l'entaille formant l'espace 42 présente deux extrémités opposées 42a, 42b qui s'étendent au-delà d'un plan médian horizontal (et au-dessus des éléments saillants 8, 9). L'écart important entre les extrémités 42a, 42b de la fente 42, plus important que le diamètre interne D facilite l'insertion de la peau scrotale 6 pour une large gamme de taille/géométrie de cette peau scrotale.

Dans l'exemple de réalisation des figures 9A et 10, il est prévu une option applicable à n'importe quel dispositif 1 conforme à l'invention, selon laquelle les reliefs 3 présentent un creux 43 et/ou sont en forme de ventouses. On obtient ainsi une très bonne adhérence, avec des reliefs 3 qui rappellent les ventouses présentes sur les bras d'une pieuvre.

Au moins pour certains des reliefs 3, la face latérale délimitant le creux 43 peut être non cylindrique, en tronc de cône qui s'évase vers la face de contact des reliefs 3.

Un avantage du dispositif 1 est sa compacité et son confort d'utilisation. Il est bien adapté pour permettre une contraception masculine, dès lors qu'il est porté de l'ordre de 14 ou 15 heures par jour environ, l'effet de contraception CMT (contraception masculine thermique) pouvant être constaté après deux mois de port régulier, de façon analogue à ce qui a été vérifié par des travaux du professeur Mieusset dans le contexte du port d'un slip remontant les testicules (Roger Mieusset et Louis Bujan : The potential of mild testicular heating as a safe, effective and réversible contraceptive method for men ; International journal of andrology ; 17 : 186-191, 1994).

Le dispositif 1 constitue, à lui seul et sans intervention médicale/chirurgicale, un outil efficace de contraception. La réversibilité de la méthode, l'absence d'effets secondaires sont en outre des avantages significatifs pour un dispositif 1 peu coûteux.

Le dispositif 1 annulaire de contraception masculine peut être produit de façon industrielle par moulage à chaud, dans un moule qui peut être typiquement annulaire. Le dispositif 1 peut être dépourvu de latex et hypoallergénique. De manière préférée, il ne contient ni colorants, ni bisphénol A, ni composés phtalates, ni agents de blanchiment, ni toxines.

Le procédé de fabrication du dispositif 1 peut utiliser un matériau polymère, par exemple thermodurcissable, pour obtenir un effet de mémoire de forme, ce matériau souple M étant de préférence à base de silicone. On moule en forme d'anneau, à chaud, le matériau polymère thermodurcissable dans une cavité annulaire du moule, en formant les reliefs 3 de la face interne F1 par moulage dans des empreintes creuses correspondantes du moule. De telles empreintes sont situées en regard d'une partie périphérique externe du moule. Eventuellement, la face externe peut être formée lisse, sans besoin d'empreintes spéciales sur la partie périphérique externe du moule.

Le moulage est typiquement un moulage par injection. Après un refroidissement du matériau moulé, on obtient le dispositif 1 qui est solide et présente une dureté relativement faible, le matériau M étant souple et étirable.

Une ou plusieurs étapes de traitement de surface peuvent être réalisées, par exemple après la solidification. La face interne F1 et la face externe F2 peuvent ainsi être rendues plus douces au contact.

Dans une variante de réalisation, le dispositif 1 peut être obtenu par une technique de fabrication additive. Les imprimantes 3D de la société Wacker conviennent par exemple pour utiliser du silicone en tant que matériau d'impression 3D.

Dans ce cas, des instructions d'impression sont fournies à partie d'un fichier représentatif de coordonnées spatiales du dispositif 1 (coordonnées dans un référentiel typiquement cartésien). L'injecteur de silicone est ensuite déplacé suivant une routine pour reconstituer les couches successives permettant de former le dispositif 1. Dans le cas d'un matériau souple M de type silicone, celui-ci peut être déposé goutte par goutte avec une viscosité élevée. Le support ou lit d'impression peut être un support en verre. A la fin du dépôt de chaque couche, les gouttelettes sont fusionnées par vulcanisation avec une source UV. Ce mode de fabrication peut être bien adapté lorsqu'il existe un besoin de réaliser des petites séries ou des prototypes.

Concernant la forme précise, le diamètre D ou dimension analogue, la position de points de contention, les valeurs ou informations fournies dans tout ce qui précède sont précisées seulement de manière non limitative pour constituer le dispositif 1.

Dans une variante de réalisation, visible sur la figure 7, le corps 15 de l'anneau présente une échancrure 22 pour le passage de la peau scrotale 16, du côté du bord avant 10b. L'échancrure 22 s'étend par exemple, d'avant en arrière, sur une distance inférieure ou égale à 10 mm, de préférence inférieure ou égale à 5 ou 6 mm. Une projection 23 peut en outre être formée sur le bord arrière 10a du côté inférieur, afin d'éviter une réduction trop importante de la portion de surface pour le contact scrotal 33.

Par ailleurs, il peut être prévu d'accessoiriser le dispositif 1 avec un appendice, par exemple un appendice vibrant ou non pour le clitoris. L'appendice est soit intégralement formé avec le corps 15 en étant joint à la face externe F2, soit attaché de façon amovible sur un organe d'attache approprié formé sur la face externe (du côté inférieur du dispositif).

Dans certaines options, le dispositif 1 peut former un objet connecté ou disposer d'une minuterie permettant de déterminer le temps de port. Eventuellement, grâce à un accessoire électronique de communication, l'utilisateur peut relier/associer le dispositif 1 à une application, typiquement une application téléchargeable par le réseau Internet, afin de gérer sa contraception (durée, efficacité, temps de pause, forum d'échange sur les pratiques, etc.).

Le dispositif 1 de contraception (maîtrise de la fertilité) se passe avantageusement de zone de chauffage active : la position supra scrotale des testicules T, au contact de la chaleur corporelle, suffit à obtenir l'effet souhaité. C'est donc un dispositif 1 à température constante auto-chauffant à usage personnel.

Ainsi, bien que les exemples illustrés montrent un dispositif 1 sous la forme d'un anneau réalisé d'une pièce ou constamment fermé, le dispositif 1 peut le cas échéant former une boucle qui peut s'ouvrir. Dans une option, la conformation en boucle fermée est permise avec un ajustement en longueur du périmètre interne du dispositif annulaire.

Le dispositif 1 peut se présenter optionnellement sous la forme d'une ceinture réglable. Alternativement, il peut être prévu une charnière élastique et des moyens de fermeture (incluant des éléments mécaniques, d'encliquetage, organes auto-agrippant, éléments magnétiques, autres éléments similaire ou combinaison de ces moyens), avec typiquement un verrouillage de la conformation en boucle du dispositif 1.

Eventuellement, on peut avoir recours à une pièce de plastique dont la charnière présente un caractère bistable, ce qui peut permettre de se passer de moyens de fermeture ou de limiter le contact de fermeture à une superposition entre les extrémités du dispositif.

## Revendications

1. Dispositif (1) de contraception masculine adapté pour encercler une verge (2), étant de forme générale annulaire autour d'un axe longitudinal (X) virtuel, le dispositif (1) comprenant :
- deux bords annulaires (10a, 10b) formant deux extrémités axiales opposées du dispositif, un premier bord dit arrière (10a) de ces deux bords étant adapté pour venir buter du côté de la racine de la verge à l'état monté du dispositif avec la verge (2) encerclée ;
- une face externe (F2) ; **caractérisé en ce que**:
- une face interne (F1) délimitant un volume intérieur (V) et présentant des reliefs (3) pour former une surface interne d'adhérence (30) du dispositif, la face interne (F1) comprenant une portion de surface supérieure (21), préférentiellement concave, destinée à être en contact avec la verge du côté de sa veine dorsale superficielle ;
des zones interstitielles s'étendant entre les reliefs (3) pour faciliter la sudation,
et **en ce que** le dispositif comporte en outre une portion de surface pour le contact scrotal (33, 133 ; 233) adaptée pour être espacée de la verge et située à l'opposé de la portion de surface supérieure (21), de sorte qu'elle permet de maintenir une partie de peau scrotale (16) entre la verge (2) placée dans le volume intérieur (V) et ladite portion de surface (33, 133 ; 233), tandis que ledit bord arrière (10a) repousse les testicules (T) en arrière du volume intérieur (V),
sachant que la portion de surface (33, 133 ; 233) est formée au moins pour partie par ladite surface interne d'adhérence (30) et/ou par des moyens de retenue formant une fente (41 ; 42) qui s'étend sous la surface interne d'adhérence (30).

2. Dispositif selon la revendication 1, dans lequel au moins huit des reliefs appartiennent à une même trajectoire circulaire dans une coupe transversale dudit dispositif, sur une moitié pour le contact avec la peau scrotale.

3. Dispositif selon la revendication 1 ou 2, dans lequel la face interne (F1) présente une conformation annulaire permettant de définir un passage traversant (5) dont l'extension radiale (D) mesurée perpendiculairement à l'axe longitudinal (X) est au minimum de 27 mm et au maximum de 53 mm dans un état non déformé, le passage traversant (5) délimité par la face interne (F1) étant apte à être déformé par déformation élastique d'un matériau souple (M) constitutif du dispositif, la distance (L15) entre les deux bords annulaires (10a, 10b) étant de préférence comprise entre 15 et 24 mm au moins du côté de ladite portion de surface pour le contact scrotal (33, 133 ; 233).

4. Dispositif selon la revendication 1, 2 ou 3, dans lequel, les reliefs (3) sont sous la forme de bossages, le dispositif (1) comprenant un matériau souple (M) polymérique pour définir les bossages.

5. Dispositif selon l'une quelconque des revendications précédentes, dans lequel la portion de surface pour le contact scrotal (33, 133 ; 233) correspond à une surface d'au moins 2 cm² et présente des premiers reliefs (3a) qui font saillie radialement vers l'intérieur.

6. Dispositif selon l'une quelconque des revendications 1 à 5, se présentant sous la forme d'un anneau réalisé d'une seule pièce, en matière élastomère, de préférence un élastomère à base de silicone.

7. Dispositif selon l'une quelconque des revendications 1 à 6, comprenant une structure à au moins deux couches dans une partie inférieure du dispositif (1), les reliefs (3) de la face interne (F1) étant au moins en partie formés par une couche dite intérieure définissant un revêtement, ladite fente (42) étant formée entre deux couches en s'étendant sous ladite couche intérieure.

8. Dispositif selon l'une quelconque des revendications précédentes, dans lequel la face interne (F1) présente, du côté de la portion de surface supérieure (21), l'un au moins parmi :
- une extension axiale (F8, F9 ; F17) décalée axialement vers l'avant par rapport au reste de la face interne (F1) ; et
- deux projections (7a, 7b) espacées entre elles d'au moins 5 mm et placées en regard l'une de l'autre, de part et d'autre d'un plan médian virtuel (P) du dispositif (1), les deux projections (7a, 7b) étant radialement saillantes vers l'intérieur.

9. Dispositif selon l'une quelconque des revendications précédentes, dans lequel les moyens de retenue formant une fente (41 ; 42) font partie d'une pièce annulaire constituant le dispositif.

10. Dispositif selon la revendication 9, dans lequel les moyens de retenue délimitent un logement, distinct du volume intérieur (V), qui est formé intérieurement dans une partie inférieure du dispositif afin recevoir la partie de peau scrotale (16), le logement étant délimité par deux faces en regard qui présentent des reliefs de retenue.

11. Dispositif selon la revendication 9 ou 10, dans lequel la fente (41) est formée transversalement à l'axe longitudinal (X) en ayant un premier débouché dans la face interne (F1) et un deuxième débouché, opposé au premier débouché, dans la face externe (F2).

12. Dispositif selon l'une quelconque des revendications précédentes, dans lequel la face interne (F1) définit un diamètre interne (D) mesuré perpendiculairement à l'axe longitudinal (X), ladite fente (41 ; 42) s'étendant entre deux extrémités (41a, 41b ; 42a, 42b) espacées entre elles d'une distance supérieure au diamètre interne (D).

13. Procédé de fabrication du dispositif (1) de contraception masculine selon l'une quelconque des revendications 1 à 12 par utilisation d'un moule, le procédé comprenant les étapes consistant essentiellement à :
- fournir un matériau polymère thermodurcissable, de préférence à base de silicone,
- mouler en forme d'anneau, à chaud, le matériau polymère thermodurcissable dans une cavité annulaire du moule, en formant les reliefs de ladite face interne par moulage dans des empreintes creuses correspondantes du moule situées en regard d'une partie périphérique externe du moule, et
- refroidir ledit matériau pour permettre son durcissement et obtenir un état solide et souple du dispositif.

## Patentansprüche

1. Vorrichtung (1) zur Empfängnisverhütung beim Mann, die dazu ausgebildet ist, einen Penis (2) zu umschließen, wobei die Vorrichtung (1) eine allgemein ringförmige Form um eine virtuelle Längsachse (X) aufweist, wobei die Vorrichtung (1) umfasst:
- zwei ringförmige Ränder (10a, 10b), die zwei gegenüberliegende axiale Enden der Vorrichtung bilden, wobei ein erster, sogenannter hinterer Rand (10a) dieser beiden Ränder dazu ausgebildet ist, im montierten Zustand der Vorrichtung mit dem umschlossenen Penis (2) an der Seite der Peniswurzel anzuliegen;
- eine Außenseite (F2); **dadurch gekennzeichnet, dass**:
- eine Innenseite (F1) ein inneres Volumen (V) begrenzt und Erhebungen (3) aufweist, um eine innere Haftfläche (30) der Vorrichtung zu bilden, wobei die Innenseite (F1) einen oberen, vorzugsweise konkaven Oberflächenabschnitt (21) umfasst, der mit dem Penis auf der Seite seiner oberflächlichen Rückenvene in Kontakt kommt;
wobei sich Zwischenraumbereiche zwischen den Erhebungen (3) erstrecken, um das Schwitzen zu erleichtern,
und dass die Vorrichtung ferner einen Oberflächenabschnitt für den Kontakt mit dem Skrotum (33, 133; 233) umfasst, der so ausgebildet ist, dass er zum Penis beabstandet ist und sich gegenüber dem oberen Oberflächenabschnitt (21) befindet, so dass er erlaubt, einen Teil der Skrotalhaut (16) zwischen dem im Innenraum (V) platzierten Penis (2) und dem Oberflächenabschnitt (33, 133; 233) zu halten, während der hintere Rand (10a) die Hoden (T) hinter das Innenvolumen (V) drückt,
wobei der Oberflächenabschnitt (33, 133; 233) wenigstens teilweise durch die innere Haftfläche (30) und/oder durch Haltemittel gebildet ist, die einen Schlitz (41; 42) bilden, der sich unter der inneren Haftfläche (30) erstreckt.

2. Vorrichtung nach Anspruch 1, wobei wenigstens acht der Erhebungen in einem Querschnitt der Vorrichtung auf einer Hälfte für den Kontakt mit der Skrotalhaut zu einer gleichen Kreisbahn gehören.

3. Vorrichtung nach Anspruch 1 oder 2, wobei die Innenseite (F1) eine ringförmige Formgebung aufweist, die es erlaubt, einen Durchgang (5) zu definieren, dessen radiale Ausdehnung (D), gemessen senkrecht zur Längsachse (X), in einem nicht verformten Zustand wenigstens 27 mm und höchstens 53 mm beträgt, wobei der von der Innenseite (F1) begrenzte Durchgang (5) durch elastische Verformung eines weichen Materials (M), das Bestandteil der Vorrichtung ist, verformt werden kann, wobei der Abstand (L15) zwischen den beiden ringförmigen Rändern (10a, 10b) bevorzugt zwischen 15 und 24 mm wenigstens auf der Seite des Oberflächenabschnitts für den Kontakt zum Skrotum (33, 133 ; 233) liegt.

4. Vorrichtung nach Anspruch 1, 2 oder 3, wobei die Erhebungen (3) in Form von Erhöhungen vorliegen, wobei die Vorrichtung (1) ein flexibles polymeres Material (M) zum Definieren der Erhöhungen umfasst.

5. Vorrichtung nach einem der vorhergehenden Ansprüche, wobei der Oberflächenabschnitt für den Kontakt zum Skrotum (33, 133; 233) einer Fläche von wenigstens 2 cm² entspricht und erste Erhebungen (3a) aufweist, die radial nach innen vorstehen.

6. Vorrichtung nach einem der Ansprüche 1 bis 5 in Form eines einteiligen Rings aus elastomerem Material, vorzugsweise einem Elastomer auf Silikonbasis.

7. Vorrichtung nach einem der Ansprüche 1 bis 6, mit einer Struktur aus wenigstens zwei Schichten in einem inneren Teil der Vorrichtung (1), wobei die Erhebungen (3) der Innenseite (Ft) wenigstens teilweise durch eine sogenannte innere Schicht gebildet werden, die eine Beschichtung definiert, wobei der Schlitz (42) zwischen zwei Schichten gebildet ist, wobei er sich unter der inneren Schicht ausdehnt.

8. Vorrichtung nach einem der vorhergehenden Ansprüche, wobei die Innenseite (Ft) auf der Seite des oberen Oberflächenabschnitts (21) wenigstens eines aus Folgendem aufweist:
- eine axiale Erstreckung (F8, F9; F17), die in Bezug auf den Rest der Innenfläche (F1) axial nach vorne versetzt ist; und
- zwei Vorsprünge (7a, 7b), die wenigstens 5 mm voneinander entfernt und einander gegenüberliegend auf beiden Seiten einer virtuellen Mittelebene (P) der Vorrichtung (1) angeordnet sind, wobei die beiden Vorsprünge (7a, 7b) radial nach innen vorstehen.

9. Vorrichtung nach einem der vorhergehenden Ansprüche, wobei die einen Schlitz bildenden Haltemittel (41; 42) Teil eines ringförmigen Teils sind, das die Vorrichtung bildet.

10. Vorrichtung nach Anspruch 9, wobei die Haltemittel eine Aufnahme begrenzen, die sich vom Innenvolumen (V) unterscheidet, das innen in einem unteren Teil der Vorrichtung zur Aufnahme des Skrotalhautteils (16) ausgebildet ist, wobei die Aufnahme durch zwei einander zugewandte Flächen begrenzt ist, die Halteerhebungen aufweisen.

11. Vorrichtung nach Anspruch 9 oder 10, wobei der Schlitz (41) quer zur Längsachse (X) mit einer ersten Mündung in der Innenseite (F1) und einer zweiten Mündung, die der ersten Mündung entgegengesetzt ist, in der Außenseite (F2) geformt ist.

12. Vorrichtung nach einem der vorhergehenden Ansprüche, wobei die Innenseite (F1) einen Innendurchmesser (D) definiert, der senkrecht zur Längsachse (X) gemessen ist, wobei sich der Schlitz (41; 42) zwischen zwei Enden (41a, 41b; 42a, 42b) erstreckt, die um einen Abstand voneinander entfernt sind, der größer als der Innendurchmesser (D) ist.

13. Verfahren zur Herstellung der Vorrichtung (1) zur Empfängnisverhütung beim Mann nach einem der Ansprüche 1 bis 12 durch Verwendung einer Form, wobei das Verfahren die Schritte umfasst, die im Wesentlichen aus Folgendem bestehen:
- Bereitstellen eines wärmehärtenden polymeren Materials, vorzugsweise auf Silikonbasis,
- ringförmiges Formen des wärmehärtenden polymeren Materials in einem ringförmigen Hohlraum der Form unter Hitze, wobei die Erhebungen der Innenseite durch Formen in entsprechenden hohlen Vertiefungen der Form, die einem äußeren peripheren Teil der Form gegenüberliegen, gebildet werden, und
- Abkühlen des Materials, um seine Aushärtung zu ermöglichen und einen festen und flexiblen Zustand der Vorrichtung zu erhalten.

## Claims

1. A male contraceptive device (1) adapted to encircle a shaft (2), being of a generally annular shape around a virtual longitudinal axis (X), the device (1) comprising:
- two annular edges (10a, 10b) forming two opposite axial ends of the device, a first edge called rear edge (10a) of these two edges being adapted to abut on the side of the shaft root in the mounted state of the device with the shaft (2) encircled;
- an outer face (F2);
**characterized in that**:
- an inner face (F1) delimiting an inner volume (V) and having protrusions (3) forms an inner grip surface (30) of the device, the inner face (F1) comprising an upper surface portion (21), preferably concave, intended to be in contact with the shaft on the side of the superficial dorsal vein thereof;
interstitial zones extending between the protrusions (3) to facilitate perspiration;
and **in that** the device further comprises a surface portion (33, 133; 233) for scrotal contact, adapted to be spaced from the shaft and located opposite the upper surface portion (21), such that it enables to keep a scrotal skin portion (16) between the shaft (2) placed in the inner volume (V) and said surface portion (33, 133; 233), whereas said rear edge (10a) pushes back the testicles (T) behind the inner volume (V),
knowing that the surface portion (33, 133; 233) is formed at least in part by said inner grip surface (30) and/or by retaining means forming a slit (41; 42) which extends under the inner grip surface (30).

2. The device according to claim 1, wherein at least eight of the protrusions (3) belong to a single circular trajectory in a transverse section of the annular device, over a half for contact with scrotal skin.

3. The device according to claim 1 or 2, wherein the inner face (F1) has an annular configuration enabling to define a through passage (5) whose radial extension (D) measured perpendicularly to the longitudinal axis (X), is at least 27 mm and at most 53 mm in an undeformed state, the through passage (5) delimited by the inner face (F1) is deformable by elastic deformation of a flexible material (M) forming the device, the distance (L15) between the two annular edges (10a, 10b) is preferably comprised between 15 and 24 mm at least on the side of said surface portion (33, 133; 233) for scrotal contact.

4. The device according to claim 1, 2 or 3, wherein the protrusions (3) have the shape of nubs, the device (1) comprising a flexible polymer material (M) to define the nubs.

5. The device according to any one of the preceding claims, wherein the surface portion (33, 133; 233) for scrotal contact corresponds to a surface area of at least 2 cm² and has first protrusions (3a) which project radially inward.

6. The device according to any one of claims 1 to 5, presenting itself in the form of a ring made as a single-piece, in an elastomer, preferably a silicone-based elastomer.

7. The device according to any one of claims 1 to 6, comprising a structure with at least two layers in a lower part of the device (1), the protrusions (3) of the inner face (F1) being at least in part formed by a layer referred as inner layer defining a coating, said slit (42) being formed between two layers extending under said inner layer.

8. The device according to any one of the preceding claims, wherein the inner face (F1) has, on the side of the upper surface portion (21), at least one among:
- an axial extension (F8, F9; F17) axially offset forward relative to the remainder of the inner face (F1); and
- two projections (7a, 7b) spaced at least 5 mm apart and facing each other, on either side of a virtual median plane (P) of the device (1), where the two projections (7a, 7b) project radially inward.

9. The device according to any one of the preceding claims, wherein the retaining means forming a slit (41; 42) are part of an annular piece constituting the device.

10. The device according to claim 9, wherein the retaining means delimit a housing, distinct from the inner volume (V), which is formed internally in a lower part of the device in order to receive the scrotal skin portion (16), the housing being delimited by two facing surfaces which present retaining reliefs.

11. The device according to claim 9 or 10, wherein the slit (41) is formed transversely to the longitudinal axis (X) by having a first opening in the inner face (F1) and a second opening, opposite the first opening, in the outer face (F2).

12. The device according to any one of the preceding claims, wherein the inner face (F1) defines an inner diameter (D) measured perpendicularly to the longitudinal axis (X), where the slit (41; 42) extends between two ends (41a, 41b; 42a, 42b) separated from each other by a distance greater than the inner diameter (D).

13. A process for manufacturing the male contraceptive device according to any one of claims 1 to 12 by using a mold, the process comprising the steps consisting essentially of:
- providing a thermoset polymer, preferably silicon-based;
- hot molding the thermoset polymer in ring shape in an annular cavity of the mold, while forming protrusions on said inner face by molding in corresponding hollow imprints of the mold located to face an outer peripheral part of the mold; and
- cooling said material in order to allow hardening thereof and obtaining a solid and flexible state of the device.
